# EUROPEAN PATENT APPLICATION

(11) **EP 0 900 567 A2**
(43) Date of publication of application: **10.03.1999**
(21) Application number: 98306661.4
(22) Date of filing: 20.08.1998
(51) Int. Cl.: A61K 31/505

(54) **Quinazoline-4-one AMPA antagonists for the treatment of dyskinesias associated with dopamine agonist therapy**

(30) Priority: 05.09.1997 US 57965 P
(71) Applicant: Pfizer Products Inc., Groton, CT 06340-5146 (US); Greenamyre, John Timothy, Atlanta, Georgia 30342 (US)
(72) Inventor: Chenard, Bertrand Leo, Waterford, Connecticut 06385 (US); Greenamyre, John Timothy, Atlanta, Georgia 30342 (US); Menniti, Frank Samuel, Mystic, Connecticut 06355 (US); Welch, Willard McKowan, Jr., Mystic, Connecticut 06355 (US)
(74) Representative: Simpson, Alison Elizabeth Fraser

(57) **Abstract**

The invention relates to a method of treating dyskinesias associated with dopamine agonist therapy in a mammal which comprises administering to said mammal an effective amount of an antagonist of the AMPA receptor. Dopamine agonist therapy, as referred to in the present invention, is generally used in the treatment of a central nervous system disorder such as Parkinson's disease.

## Description

### Background Of The Invention

This invention relates to a method of administering an AMPA receptor antagonist to treat dyskinesias in mammals, such as humans, resulting from the use of dopamine agonist therapy. Dopamine agonist therapy, as referred to in the present invention, is generally used in the treatment of a central nervous system disorder such as Parkinson's disease.

Dyskinesias are involuntary physical movements which may include chorea, tremor, ballism, dystonia, athetosis, myoclonus and tic. Dyskinesias often result from treatment of the physical symptoms of Parkinson's disease. Parkinson's disease is characterized by tremor, rigidity, bradykinesia and postural instability. Such motor abnormalities may be reduced by therapies which increase dopamine receptor stimulation. These therapies include drugs which directly stimulate dopamine receptors (such as bromocriptine) or increase the levels of dopamine (such as L-dopa or drugs which inhibit dopamine metabolism). In the present invention, such therapies which increase dopamine receptor stimulation are referred to generally as dopamine agonist therapy. After a period of chronic administration of dopamine agonist therapy to treat Parkinson's disease, new motor abnormalities may emerge. The motor abnormalities associated with dopamine agonist therapy include choreatic dyskinesias and dystonias. The present invention relates to the treatment of dyskinesias associated with dopamine agonist therapy in the treatment of a central nervous system (CNS) disorder, in particular Parkinson's disease, through the administration of an AMPA receptor antagonist.

The agent that may be used in accord with the present invention is an antagonist of the AMPA subtype of the glutamate receptor. Glutamate is the principal excitatory neurotransmitter in the central nervous system of mammals. Glutamate synaptic transmission is mediated by several families of receptors including the α-amino-3-hydroxy-5-methyl-4-isoxazolepropionic acid (AMPA), N-methyl-D-aspartate (NMDA), kainic acid (KA), and metabotropic receptors. The AMPA receptor subtype mediates fast excitatory transmission throughout the brain, including areas involved in movement. By inhibiting the AMPA receptor through administration of an AMPA receptor antagonist, dyskinesias associated with dopamine agonist therapy may be treated in accord with the present invention as described below.

AMPA receptor antagonists are referred to in several published patents including the following issued United States patents (listed by patent number followed by issue date in parentheses): 5,654,303 (August 5, 1997); 5,639,751 (June 17, 1997); 5,614,532 (March 25, 1997); 5,614,508 (March 25, 1997); 5,606,062 (February 25, 1997); 5,580,877 (December 3, 1996); 5,559,125 (September 24, 1996); 5,559,106 (September 24, 1996); 5,532,236 (July 2, 1996); 5,527,810 (June 18, 1996); 5,521,174 (May 28, 1996); 5,519,019 (May 21, 1996); 5,514,680 (May 7, 1996); 5,631,373 (May 20, 1997); 5,622,952 (April 22, 1997); 5,620,979 (April 15, 1997); 5,510,338 (April 23, 1996); 5,504,085 (April 2, 1996); 5,475,008 (December 12, 1995); 5,446,051 (August 29, 1995); 5,426,106 (June 20, 1995); 5,420,155 (May 30, 1995); 5,407,935 (April 18, 1995); 5,399,696 (March 21, 1995); 5,395,827 (March 7, 1995); 5,376,748 (December 27, 1994); 5,364,876 (November 15, 1994); 5,356,902 (October 18, 1994); 5,342,946 (August 30, 1994); 5,268,378 (December 7, 1993); and 5,252,584 (October 12, 1993).

### Summary Of The Invention

This invention relates to a method of treating dyskinesias associated with dopamine agonist therapy in a mammal, such as a human, which comprises administering to said mammal an amount of an AMPA receptor antagonist that is effective in treating said dyskinesia.

In a specific embodiment of the above method, said dopamine agonist therapy is therapy comprising the administration of L-dopa or L-dopa in combination with an inhibitor of peripheral dopadecarboxylase such as carbidopa or benserazide.

In another specific embodiment of the above method, said AMPA receptor antagonist is 3-(2-chloro-phenyl)-2-[2-(6-diethylaminomethyl-pyridin-2-yl)-vinyl]-6-fluoro-3H-quinazolin-4-one or a pharmaceutically acceptable salt thereof.

This invention also relates to a method of treating dyskinesias associated with dopamine agonist therapy in a mammal, such as a human, which comprises administering to said mammal an AMPA receptor antagonizing effective amount of an AMPA receptor antagonist.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, refers to the act of treating, as "treating" is defined immediately above.

The term "dyskinesia(s)", as used herein, unless otherwise indicated, means any abnormal or uncontrollable movement including, but not limited to, chorea, tremor, ballism, dystonia, athetosis, myoclonus and tic.

The term or phrase "dopamine agonist therapy", as used herein, unless otherwise indicated, means any therapy that increases dopamine receptor stimulation, including, but not limited to, therapies that directly stimulate dopamine receptors (such as bromocriptine) and therapies that increase the levels of dopamine (such as L-dopa or drugs which inhibit dopamine metabolism). Dopamine agonist therapies include, but are not limited to, therapies which comprise the administration of one or more of the following agents: L-dopa, L-dopa in combination with an L-dopa decarboxylase inhibitor such as carbidopa or benserazide, bromocriptine, dihydroergocryptine, etisulergine, AF-14, alaptide, pergolide, piribedil, dopamine D1 receptor agonists such as A-68939, A-77636, dihydrexine, and SKF-38393; dopamine D2 receptor agonists such as carbergoline, lisuride, N-0434, naxagolide, PD-118440, pramipexole, quinpirole and ropinirole; dopamine/β-adrenergic receptor agonists such as DPDMS and dopexamine; dopamine/5-HT uptake inhibitor/5-HT-1A agonists such as roxindole; dopamine/opiate receptor agonists such as NIH-10494; α2-adrenergic antagonist/dopamine agonists such as terguride; α2-adrenergic antagonist/dopamine D2 agonists such as ergolines and talipexole; dopamine uptake inhibitors such as GBR-12909, GBR-13069, GYKI-52895, and NS-2141; monoamine oxidase-B inhibitors such as selegiline, N-(2-butyl)-N-methylpropargylamine, N-methyl-N-(2-pentyl)propargylamine, AGN-1133, ergot derivatives, lazabemide, LU-53439, MD-280040 and mofegiline; and COMT inhibitors such as CGP-28014, entacapone and tolcapone. Dopamine agonist therapy, as referred to in the present invention, is used in the treatment of a central nervous system disorder such as, but not limited to, Parkinson's disease.

The term or phrase "dyskinesia associated with dopamine agonist therapy", as used herein, unless otherwise indicated, means any dyskinesia which accompanies, or follows in the course of, dopamine agonist therapy, or which is caused by, related to, or exacerbated by dopamine agonist therapy, wherein dyskinesia and dopamine agonist therapy are as defined above.

The method of the present invention also relates to the use of pharmaceutically acceptable acid addition salts of an AMPA receptor antagonist. The acids which are used to prepare the pharmaceutically acceptable acid addition salts of the aforementioned AMPA receptor antagonist are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3- naphthoate)]salts.

The invention also relates to the use of base addition salts of an AMPA receptor antagonist. The chemical bases that may be used as reagents to prepare pharmaceutically acceptable base salts of said AMPA receptor antagonist that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine (meglumine), and the lower alkanolammonium and other base salts of pharmaceutically acceptable organic amines.

### Detailed Description Of The Invention

The method of the present invention is readily practiced by those skilled in the art. In its broadest scope, the method of the present invention comprises the use of any AMPA antagonist to treat dyskinesia associated with dopamine agonist therapy. Various AMPA receptor antagonists are familiar to those skilled in the art including the AMPA receptor antagonists referred to in the issued United States patents listed above in the Background Of The Invention.

In a specific embodiment of the present invention, the method comprises the administration of 3-(2-chloro-phenyl)-2-[2-(6-diethylaminomethyl-pyridin-2-yl)-vinyl]-6-fluoro-3H-quinazolin-4-one, or a pharmaceutically acceptable salt thereof, to a mammal to treat dyskinesias associated with dopamine agonist therapy. The foregoing compound, which is an AMPA receptor antagonist, may be prepared as described below.

### 3-(2-Chlorophenyl)-2-[2-(6-diethylaminomethylpyridin-2-yl)-vinyl]-6-fluoro-3H-quinazolin-4-one

### Method A

### 6-Fluoro-2-methylquinoxalin-4-one

A solution of 12.95 g (70.0 mmol) of 2-nitro-5-fluorobenzoic acid in 200 mL of glacial acetic acid and 20 mL of acetic anhydride was treated with 0.625 g of 10% palladium on carbon are reduced at an initial pressure of 54.5 psi. Hydrogen uptake was complete after two hours. The catalyst was removed by filtration and the filtrate was heated at reflux for two hours at which time TLC (1:1 hexane/ethyl acetate) indicated that the reaction was complete. The reaction mixture was evaporated to a semicrystalline mass which was broken up in a minimum amount of 2-propanol and stirred in an ice bath for one hour. The crystalline solid was separated by filtration, washed with minimal cold 2-propanol and air dried to give 5.79 g (46%) of the desired product as a brown solid, m.p. 127.5 - 128.5°C.

A synthesis of 5-fluoro-2-nitrobenzoic acid is described by Slothouwer, J. H., Recl. Trav. Chim. Pays-Bas. 33, 336 (1914).

### Method B

### 3-(2-Chlorophenyl)-6-fluoro-2-methyl-4-(3H)-quinazolinone

A solution of 2.50 g (14.0 mmol) of 6-fluoro-2-methylquinoxalin-4-one and 1.96 g (15.4 mmol) of 2-chloroaniline in about 20 mL of glacial acetic acid was heated at reflux under a nitrogen atmosphere for 6 hours. Most of the solvent was evaporated from the cooled reaction mixture and the residues were taken up in ethanol and refrigerated. After 6 days in the refrigerator, the formed crystals were filtered off, washed with minimal cold ethanol and air dried to give 1.79 g (44%) of the product. m.p. 137-138°C.

### Method C

### 6-(2-[3-(2-Chlorophenyl)-6-fluoro-4-oxo-3,4-dihydroquinazolin-2-yl-vinyl)pyridine-2-carbaldehyde

A catalytic amount (about 100 mg) of anhydrous zinc chloride was added to a solution of 576 mg (2.0 mmol) of 3-(2-chlorophenyl)-6-fluoro-2-methyl-4(3H)-quinazolinone and 270 mg (2.0 mmol) of 2,6-pyridinedicarboxaldehyde in 20-25 mL of dioxane and 1.0 mL of acetic anhydride. The reaction mixture was heated at reflux under a nitrogen atmosphere for 3 hours until TLC indicated that the starting materials had been consumed. The cooled reaction mixture was poured into water and the mixture was extracted with ethyl acetate. The combined extracts were dried with brine and magnesium sulfate, treated with decolonizing carbon and filtered and the solvent was removed to give the desired product. This was taken up in 2:1 ether/pentane and the crystals were filtered to give 266 mg of the product, 33%, m.p. 247-248°C.

A synthesis of pyridine-2,6-dicarboxaldehyde is described by Papadopoulos, et. al., J. Org. chem., 31 , 615(1966).

### Method D

### 3-(2-Chlorophenyl)-2-[2-(6-diethylaminomethylpyridin-2-yl)-vinyl]-6-fluoro-3H-quinazolin-4-one

A solution of 65 mg (0.16 mmol) of 6-{2-[3-(2-chlorophenyl)-6-fluoro-4-oxo-3,4-dihydroquinazolin-2-yl)-vinyl)pyridine-2-carbaldehyde in 10 mL of methylene chloride at room temperature under a nitrogen atmosphere was treated with 3 drops of diethylamine and 73 mg (0.34 mmol) of sodium triacetoxyborohydride. After stirring for 2 1/2 hour at room temperature, the solvent was evaporated and the residues were partitioned between dilute hydrochloric acid and either and stirred for 30 minutes. The ethereal layer was separated and the aqueous was extracted once again with either; the ethereal extracts were discarded. The aqueous acidic solution was adjusted to pH = 14 with 10% sodium hydroxide (ice bath cooling) and was then extracted with either twice. The combined ethereal extracted were dried with brine and with magnesium sulfate and the solvent was evaporated. After one attempt to form a mesylate salt, the reworked free base in ethyl acetate was treated with 7.5 mg (0.06 mmol) of maleic acid dissolved in a little ethyl acetate. Crystals formed from the resulting solutions which were filtered and washed with ethyl acetate to give 22 mg of the monomaleate salt, (24%), m.p. 170.5-171.5°C,

An AMPA receptor antagonist to be used in the present invention which is basic in nature is capable of forming a wide variety of different salts with various inorganic and organic acids. Although such salts must be pharmaceutically acceptable for administration to animals, it is often desirable in practice to initially isolate said AMPA receptor antagonist from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of the method of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

The acids which are used to prepare a pharmaceutically acceptable acid addition salt of an AMPA receptor antagonist to be used in the present invention are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts.

Those AMPA receptor antagonists to be used in the present invention which are acidic in nature are capable of forming base salts with various pharmacologically acceptable cations. Examples of such salts include the alkali metal or alkaline-earth metal salts and particular, the sodium and potassium salts. These salts are all prepared by conventional techniques. The chemical bases which are used as reagents to prepare the pharmaceutically acceptable base salts of this invention are those which form non-toxic base salts with the AMPA receptor antagonists to be used in the present invention. These non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction of maximum product of yields of the desired final product.

The *in vitro* and *in vivo* activity of a compound to be used in the present invention for AMPA receptor antagonism can be determined by methods available to one of ordinary skill in the art. One method for determining the activity of said compound is by blockage of AMPA receptor activation-induced ⁴⁵Ca²⁺ uptake into neurons. A specific method for determining blockage of AMPA receptor activation-induced ⁴⁵Ca²⁺ uptake into neurons is described below.

### Neuronal primary cultures

Primary cultures of rat cerebellar granule neurons are prepared as described by Parks, T.N., Artman, L.D., Alasti, N., and Nemeth, E.F., Modulation Of N-Methyl-D-Aspartate Receptor-Mediated Increases In Cytosolic Calcium In Cultured Rat Cerebellar Granule Cells, Brain Res. 552, 13-22 (1991). According to this method, cerebella are removed from 8 day old CD rats, minced into 1 mm pieces and incubated for 15 minutes at 37°C in calcium-magnesium free Tyrode's solution containing 0.1% trypsin. The tissue is then triturated using a fine bore Pasteur pipette. The cell suspension is plated onto poly-D-lysine coated 96-well tissue culture plates at 10⁵ cells per well. Medium consists of Minimal Essential Medium (MEM), with Earle's salts, 10% heat inactivated Fetal Bovine Serum, 2 mM L-glutamine, 21 mM glucose, Penicillin-Streptomycin (100 units per ml) and 25 mM KCI. After 24 hours, the medium is replaced with fresh medium containing 10µM cytosine arabinoside to inhibit cell division. Cultures are used 6 to 8 days later.

### AMPA receptor activation-induced ⁴⁵Ca²⁺ uptake

The effects of drugs on AMPA receptor activation-induced ⁴⁵Ca²⁺ uptake can be examined in rat cerebellar granule cell cultures prepared as described above. Cultures in 96 well plates are preincubated for approximately 3 hours in serum free medium and then for 10 minutes in a Mg²⁺-free balanced salt solution (in mM: 120 NaCI, 5 KCI, 0.33 NaH₂PO₄ 1.8 CaCl₂, 22.0 glucose and 10.0 HEPES at pH 7.4) containing 0.5 mM DTT, 10 uM glycine and drugs at 2X final concentration. The reaction is started by rapid addition of an equal volume of the balanced salt solution containing 100 µM of the AMPA receptor agonist kainic acid and ⁴⁵Ca²⁺ (final specific activity 250 Ci/mmol). After 10 minutes at 25°C, the reaction is stopped by aspirating the ⁴⁵Ca²⁺-containing solution and washing the cells 5X in an ice cold balanced salt solution containing no added calcium and 0.5 mM EDTA. Cells are then lysed by ovemight incubation in 0.1 % Triton-X100 and radioactivity in the lysate is then determined.

The following procedure may be used to assess the efficacy of an AMPA receptor antagonist in the treatment of dyskinesias associated with dopamine agonist therapy in the treatment of Parkinson's disease. Aged, female rhesus monkeys are rendered Parkinsonian as follows. Each monkey is first infused with 0.4 mg/kg MPTP (1-methyl-4-phenyl-1,2,3,6-tetrahydropyridine) via the right internal carotid artery. After being evaluated behaviorally for 3 to 6 weeks and being judged to have stable unilateral deficits, the animals receive a second MPTP injection via the left internal carotid artery. Monkeys lesioned according to this protocol have been shown to have stable, bilateral deficits that are responsive to L-dopa and apomorphine. Once the monkeys are Parkinsonian, dyskinesias are induced over a period of approximately 3 to 6 weeks by treating the monkeys twice daily with subcutaneous injections of PHNO ((+)-4-propyl-9-hydroxynaphthoxazine) (a dopamine agonist). Dyskinesias are assessed 30 minutes after PHNO injection and every 30 minutes for the next 120 minutes (5 measurements) taking into account the following: type of dyskinesia (chorea, dystonia); intensity (0 = absent; 1 = mild; 2 = moderate; 3 = severe); and topography (arm, leg, trunk, generalized). The overall score (0 - 3) is averaged over the 5 measurements. Scoring is performed blindly from coded videotapes. An AMPA receptor antagonist is then administered together with the dopamine agonist at dosages ranging from 0.05 mg/kg to 1 mg/kg.

Pharmaceutical compositions for use in the method of the present invention may be prepared according to methods familiar to those skilled in the art. For example, pharmaceutical compositions containing an AMPA receptor antagonist for use in the present invention (hereinafter an "active compound") may be formulated in a conventional manner using one or more pharmaceutically acceptable carriers. Thus, an active compound may be formulated for oral, buccal, intranasal, parenteral (e.g., intravenous, intramuscular or subcutaneous), transdermal (e.g., patch, ointment, cream or iontophoresis), or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycollate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); nonaqueous vehicles (e.g., almond oil, oily esters or ethyl alcohol); and preservatives (e.g., methyl or propyl p-hydroxybenzoates or sorbic acid).

For buccal administration, the pharmaceutical composition may take the form of tablets or lozenges formulated in conventional manner.

An active compound may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

An active compound may also be formulated in rectal compositions such as suppositories or retention enemas, e.g., containing conventional suppository bases such as cocoa butter or other glycerides.

For intranasal administration or administration by inhalation, an active compound is conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of an active compound. Capsules and cartridges (made, for example, from gelatin) for use in an inhaler or insufflator may be formulated containing a powder mix of an active compound and a suitable powder base such as lactose or starch.

A proposed dose of an active compound for use in the method of the present invention for oral, parenteral or buccal administration to the average adult human requiring treatment is 0.01 to 100 mg/kg of the active ingredient per unit dose which could be administered, for example, 1 to 4 times per day.

Aerosol formulations for use in the method of the present invention in the treatment of an average adult human are preferably arranged so that each metered dose or "puff" of aerosol contains 20µg to 1000µg of the active compound. The overall daily dose with an aerosol will be within the range 100 µg to 10 mg. Administration may be several times daily, for example 2, 3, 4 or 8 times, giving for example, 1, 2 or 3 doses each time.

For transdermal administration the composition may take the form of patches, creams, ointments or iontophoresis formulated in conventional manner such as described in United States Patents 5,004,610 and 5,364,630, issued April 2, 1991 and November 15, 1994 respectively.

## Claims

1. A method of treating dyskinesia associated with dopamine agonist therapy in a mammal which comprises administering to said mammal an amount of an AMPA receptor antagonist that is effective in treating said dyskinesia.

2. The method of claim 1 wherein said dopamine agonist therapy is therapy comprising the administration of L-dopa or L-dopa in combination with an inhibitor of peripheral dopadecarboxylase

3. The method of claim 2 wherein said inhibitor of peripheral dopadecarboxylase is carbidopa or benserazide.

4. The method of claim 1 wherein said compound is 3-(2-chloro-phenyl)-2-[2-(6-diethylaminomethyl-pyridin-2-yl)-vinyl]-6-fluoro-3H-quinazolin-4-one or a pharmaceutically acceptable salt thereof.

5. A method of treating dyskinesia associated with dopamine agonist therapy in a mammal which comprises administering to said mammal an AMPA receptor antagonizing effective amount of a compound that is an antagonist of the AMPA receptor or a pharmaceutically acceptable salt of said compound.

6. The method of claim 5 wherein said dopamine agonist therapy is therapy comprising the administration of L-dopa or L-dopa in combination with an inhibitor of peripheral dopadecarboxylase.

7. The method of claim 6 wherein said inhibitor of peripheral dopadecarboxylase is carbidopa or benserazide.

8. The method of claim 5 wherein said compound is 3-(2-chloro-phenyl)-2-[2-(6-diethylaminomethyl-pyridin-2-yl)-vinyl]-6-fluoro-3H-quinazolin-4-one or a pharmaceutically acceptable salt thereof.
